# EUROPÄISCHE PATENTSCHRIFT

(11) **EP 0 532 979 B1**
(45) Veröffentlichungstag und Bekanntmachung des Hinweises auf die Patenterteilung: **16.07.1997**
(21) Anmeldenummer: 92114992.8
(22) Anmeldetag: 02.09.1992
(51) Int. Cl.: C07K 14/00, C12P 21/08, G01N 33/577, G01N 33/68, A61K 39/395

(54) **Petide zur Herstellung von Mitteln zur Diagnose und Therapie von systemischem Lupus**
Peptides for the preparation of means for diagnosis and therapy of systemic lupus
Peptides pour la préparation de moyens pour la diagnose et la thérapie du lupus systémique

(30) Priorität: 16.09.1991 DE 4130786
(43) Veröffentlichungstag der Anmeldung: 24.03.1993
(73) Patentinhaber: SYMBIOTEC Gesellschaft zur Forschung und Entwicklung auf dem Gebiet der Biotechnologie GmbH, D-35745 Herborn (DE)
(72) Erfinder: Zeppezauer, Michael Prof. Dr., W-6601, Scheidt (DE); Schönberger, Arno Dr., W-2000, Hamburg 73 (DE); Cebecauer, Ladislav Dr., 92101 Piestany (CS)
(74) Vertreter: Pätzold, Herbert, Dr.-Ing.

(56) Entgegenhaltungen:
- GENOMICS, Bd. 10, Nr. 4 , August 1991, Seiten 940-948; ALBIG ET AL.: 'Isolation and characterization of two human H1 histone genes within clusters of core histone genes'
- J. BIOCHEM., Bd. 85, Nr. 2 , 1979, Seiten 615-624; OHE ET AL.: 'Human spleen histone H2B, isolation and aminoacid sequence'
- INT. ARCH. ALLERGY APPL. IMMUNOL., Bd. 89, Nr. 2-3 , 1989, Seiten 288-296; MULLER ET AL.: 'Reactivity of autoantibodies in systemic lupus erythematosus with synthetic core histone peptides'
- J. IMMUNOLOGY, Bd. 144, Nr. 12 , 15. Juni 1990, Seiten 4633-4640; PORTANOVA ET AL.: 'Histone autoantigens in murine lupus : definition of a major epitope within an accessible region of chromatin'
- MOL. IMMUNOL., Bd. 26, Nr. 8 , August 1989, Seiten 749-758; MONESTIER: 'Monoclonal anti-histone H1 autoantibodies from MRL lpr/lpr mice'
- AUTOIMMUNITY, Bd. 9, Nr. 1, 1991, Seiten 13-19; MINOTA ET AL.: 'Specificity of autoantibodies to histone H1 in SLE : relationship to DNA-binding domains'
- ARTHRITIS RHEUM., Bd. 33, Nr. 9SUP, 1990, Seite S51; MINOTA: 'Autoantibody epitope and DNA-binding site on histone H1'
- PROC. NATL ACAD. SCI., Bd. 80, Nr. 24, Dezember 1983, Seiten 7410-7414; HARDIN ET AL.: 'Antibodies to histones in systemic lupus erythematosus : localization of prominent autoantigens on histone H1 and H2B'
- J. MOL. EVOL., Bd. 25, Nr. 4, 1987, Seiten 361-370; TOENJES ET AL.: 'A highly conserved sequence in H1 histone genes as an oligonucleotide hybridization probe : isolation and sequence of a duck H1 gene'
- MOL. IMMUNOL., Bd. 24, Nr. 3, Mai 1987, Seiten 275-285; GOHILL ET AL.: 'Antibodies in procainamide-induced and systemic lupus erythematosus bind the C-terminus of histone 1 (H1)'
- J. IMMUNOLOGY, Bd. 133, Nr. 5, November 1984, Seiten 2554-2559; KOTZIN ET AL.: 'Monoclonal anti-histone autoantibodies derived from murine models of lupus'
- CLIN. EXP. IMMUNOL., Bd. 86, Nr. 1, Oktober 1991, Seiten 124-133; ATANASSOV ET AL.: 'New Zealand white rabbits immunized with RNA-complexed total histones develop an autoimmune-like response'

## Beschreibung

Die Erfindung bezieht sich auf Peptide mit antigenen oder immunogenen Determinanten, die von Autoantikörpern in Körperflüssigkeiten von Patienten anerkannt werden, die an systemischem Lupus erythematodes (SLE) erkrankt sind.

Die Krankheiten des "rheumatischen Formenkreises" sind charakterisiert durch eine Vielzahl klinischer Erscheinungsbilder und ein breites Spektrum von Autoantikörpern. Letztere sind gegen verschiedenartige Bestandteile normaler Zellen gerichtet. Zu den genannten Krankheiten gehört systemischer Lupus erythematodes (SLE), welcher spontan auftreten oder auch durch Arzneimittel induziert werden kann. Bei SLE treten besonders häufig Autoantikörper auf, welche gegen Bestandteile des Zellkerns gerichtet sind (antinuclear antibodies, ANA-s); dazu gehören unter anderem doppelstrangige Desoxyribonucleinsäure (DS-DNA) und Histonproteine, Ribonucleinsäure (RNA), Komplexe von DNA und Histonen sowie Enzyme. Die Histone bestehen aus mehreren Klassen von Proteinen, den sogenannten Core-Histonen H2A, H2B, H3 und H4, welche in den Nucleosomen gefunden werden, und den Linker-Histonen H1 und H5, welchen verknüpfende Funktionen beim Aufbau des Chromatins zugeschrieben werden. Man hat vielfach versucht, die Häufigkeit von Autoantikörpern, welche gegen spezielle Antigene gerichtet sind, mit bestimmten rheumatischen Krankheitsbildern zu korrelieren.

Es wurde gefunden, daß bei SLE Autoantikörper gegen Histone (AHA-s, Anti-Histon-Autoantikörper) gehäuft auftreten. Als Nachweismethode dient üblicherweise der "enzyme-linked immunsorbent assay" (ELISA), bei welchem die Seren von Patienten und gesunden Vergleichspersonen gegen gereinigte Zellbestandteile (d.h. Antigene) getestet werden. Als Antigene kamen beim Test von SLE-Seren u.a. die reinen Histone zum Einsatz.

Zusätzlich wurden als Antigene auch synthetische oder durch Abbau natürlicher Histone erhaltene Peptide verwendet, welche aus Teilsequenzen der genannten Histone bestanden.

Dabei zeigte sich, daß bei Verwendung der einzelnen Histone und Histonpeptide
(i) die Häufigkeit einer positiven Reaktion im ELISA-Test nicht höher als ca. 50 % liegt und daß
(ii) die Häufigkeit einer positiven Reaktion bei Patientenseren von anderen rheumatischen Krankheiten groß ist (falsch-positive Ergebnisse).

So wurde kürzlich in einer Studie über den prädiktiven Wert der Erkennung von AHA-s bei SLE-Patienten (mittels des LE cell test, Smeenk et al, Scand. J. Rheumatol. Suppl. 56, 78 - 92, 1985) festgestellt: Obwohl 95 % der SLE-Patienten im LE-Test positiv waren, ist die Chance, daß ein Patient mit positivem LE-Test tatsächlich SLE hat, nur 27 %.

Es besteht daher die Aufgabe, den prädiktiven Wert diagnostischer Tests für SLE zu verbessern, d.h. den Prozentsatz von echt-positiven zu falsch-positiven zu erhöhen.

Es besteht die weitere Aufgabe monoklonale Antikörper und antiidiotypische Antikörper bilden zu können, die in ihrer Spezifität den Autoantikörpern von SLE-Patienten genau entsprechen, sowie die weitere Aufgabe anti-idiotypische Antikörper (monoklonale Antikörper) bilden zu können, die gegen diese monoklonalen Antikörper bzw. die Autoantikörper von SLE-Patienten gerichtet sind.

Die Aufgabe wird erfindungsgemäß durch die Merkmale des Anspruchs 1 gelöst. Vorteilhafte Ausbildungen und Weiterbildungen ergeben sich aus den Merkmalen der weiteren Ansprüche und/oder der nachstehenden Beschreibung.

Folgende natürliche und künstliche Peptide wurden getestet:

Die Aminosäuren wurden gemäß dem "one-letter-code" abgekürzt:

GLY=G, ALA=A, VAL=V, LEU=L, ILE=I, PHE=F, PRO=P, SER=S, THR=T,
CYS=C, MET=M, TRP=W, TYR=Y, ASN=N, GLN=Q, ASP=D, GLU=E, LYS=K,
ARG=R, HIS=H.

### Histon-H1-Peptide

H1-N-Terminus : 3 - 31
A P A A P  A A A P P  A E K T P  V K K K A  A K K
P A  G A

H1: 55 - 75
R S G V S L A A L K  K A L A A  A G Y D V E

H1: 97 - 116
T K G T G  A S G S F  K L N K K  A A S G E

H1: 76 - 116
K N N S  R I K L G  L K S L V  S K G T L  V E T K G
T G A S G  S F K L N  K K A A S  G E

H1: 66 - 116
A L A A  A G Y D V  E K N N S  R I K L G  L K S L V
S K G T L  V E T K G  T G A S G  S F K L N  K K A A
S G E

H1: 55 - 166
R S G V S  L A A L K  K A L A A  A G Y D V  E K N N S
R I K L G  L K S L V  S K G T L  V E T K G  T G A S G
S F K L N K K A A S  G E

H1-C-Terminus: 187 - 211
K P K A A  K P K A A  K P K A A  K P K K A  A P K K K

### Histon H2B-Peptide

H2B: 1-35
P E P A K  S A P A P  K K G S K  K A V T K  A Q K K
D  G K K R K  R S E K E

H2B: 36-76
S Y S V Y  V Y K V L  K Q V H P  D T G I S  S K A M G
I M N S F  V N D I F  E R I A G  E

H2: 77-93
A S R L A  H Y N K R  S T I T S  R E

H2B: 94 - 105
I Q T A V  R L L L P  G E

H2B: 106-113
L A K H A  V S E

H2B: 113 - 125
G T K A V  T K Y T S  S K

H2B: N-Term.  1 - 21
P E P A K  S A P A P  K K G S K  K A V T K A

H2B N-Term: 4 - 11
A K S A P A P K

### Histon H2A-Peptid

H2A-N-Terminus
S G R G K  Q G G K A  R A K A K  T R S S R  A G

### Histonsequenzen:

H2A:

S G R G K  Q G G K A  R A K A K  T R S S R  A G L Q F
P V G R V  H R L L R  K G N Y A  E R V G A  G A P V Y
L A A V L  E Y L T A  E L L E L  A G N A A  R D N K K

T R I I P  R H L Q L  A I R N D  E E L N K  L L G K V
T I A Q G  G V L P N  I Q A V L  L P K K T  E S H H K
A K G K.

Mittels ELISA-Test wurden die Epitope der Autoantikörper von 122 Rheuma- und SLE-Seren mit H1, H2B und H2A-Peptiden kartiert. 80 % der SLE-Seren und 68% aller Seren reagierten positiv sowohl gegen den C-Terminus von H1 als auch gegen den N-Terminus von H2B. Die Kombination beider Regionen ist somit als Markersequenz und damit als Unterscheidungskriterium für SLE-Patienten anzusehen. Sowohl die strukturellen Daten über diese Regionen als auch die Antigenitätsberechnungen und die homologen Epitope der eigenen, in vivo und in vitro hergestellten Antikörper unterstreichen den dominanten antigenen Charakter des N-Terminus von H2B und des C-Terminus von H1.

Für den ELISA-Test (Enzym-linked-immuno-sorbent-assay) wurden F16-Module der Firma Nunc mit speziell hochaktivierter Oberfläche benutzt. Je nach Testansatz wurden entweder der zu testende Antikörper (direkter ELISA-Test bzw. Sandwich-Test) oder das Antigen (indirekter ELISA-Test) an die Oberfläche der Mikrotiterplatte gebunden. Die Antigene wurden in einer Konzentration von 50 µg/ml in einem 0,05 M Carbonatpuffer, pH 9,7 gelöst. Antikörperlösungen, Zellüberstände sowie Urinproben wurden 1:3 im gleichen Puffer verdünnt und je zu 100 µl auf die Mikrotiterplatte pipettiert. Die Bindung vollzog sich über 24 h bei 4°C. Nach Entleeren der Näpfe erfolgte am nächsten Tag ein einstündiges Abblocken reaktiver Gruppen der Mikrotiterplatte bei 36°C mit 250 µl Blockerlösung/Napf. Verschiedene Blockerlösungen wurden verwendet:
0,5 % (w/v) Gelatine in PBS/Azid: 1 % (w/v) BSA in PBS/Azid; 5 % (w/v) BSA in PBS/Azid; 10 % (v/v) Pferdeserum in PBS/Azid. Es erfolgte die Zugabe von 100 µl Zellkulturüberständen (primärer Antikörper) bzw. die 1:250 verdünnten Seren mit einstündiger Inkubation bei Raumtemperatur im Dunkeln. Nach einmaligem Spülen der Mikrotiterplatte mit Tweenlösung (bestehend aus 0,1 % (v/v) Tween 20 und 150 mM NaCl) wurden 100 µl Konjugat (0,3 % (v/v) Kaninchen-anti(Maus-IgG)IgG-HRP bzw. Kaninchen-anti(Human-IgG)IgG-HRP hinzupepittiert und 1 h bei Raumtemperatur inkubiert. Nicht gebundene Antikörper wurden durch fünfmaliges Spülen mit Tweenlösung entfernt. Die an den Kaninchen- bzw. Schafsantikörpern gekoppelte Meerettichperoxidase vollzog nach Zugabe von 100 µl des Mc Ilvanie Puffers (116 mM Na₂HPO₄ + 2 H₂O, 42 mM Zitronensäure, pH 5,6, einschließlich 1,5 mM Orthophenylendiamin und 0,9 mM H₂O₂) im Dunkeln die Farbreaktion mit 100 µl 2 M H₂SO₄ gestoppt. Die Extinktion der verschiedenen Näpfe wurden nach Abgleich gegen die Blindprobe photometrisch bei 490 nm mit Hilfe des Minireaders II ermittelt und die so erhaltenen Werte ausgedruckt.

Die 122 SLE-Seren wurden im ELISA-Test auf Autoantikörper getestet. Die ELISA-Testversuche zeigten, daß der N-terminale Bereich (1 - 35) von H2B und der C-terminale Bereich (187 - 211) von H1 bevorzugte Epitope von SLE-Autoantikörpern darstellen. Ebenfalls erwies sich die Verdünnungsstufe 1:250 als besonders geeignet, ein breites Spektrum hoch- und niedrigtitriger Seren im ELISA-Test zu erfassen. Das Hauptaugenmerk wurde dabei erfinderseits auf IgG-Autoantikörper gelegt.

Die Ergebnisse wurden folgendermaßen ausgewertet: Ein ELISA-Test eines Patienten war nur dann positiv, wenn beide Extinktionen > 0,2 (Cutt off = 0,2 aus Leerwertmessungen und Korrekturfaktor bei Ausreißern > 0,2) und deutlich höher waren, als die gegenüber allen anderen Peptiden.

Von den 122 Seren waren 68 % hinsichtlich der Peptidkombination positiv. Die 122 Seren teilten sich in 80 SLE und 42 Rheumaseren auf. Von den 80 Lupus-Patienten waren 80 % H1-CT und E1 positiv, während von den 42 Rheuma-Patienten noch 45 % H1-CT und E1 positiv waren. Damit stellen der N-terminale Bereich von H2B (1-31) und der C-terminale Bereich von H1 (187-211) die hauptsächlich erfaßten antigenen Determinanten von Autoantikörpern der Lupus-Patienten dar. Die Kombination dieser beiden Peptide kann somit als Unterscheidungskriterium zur Klassifizierung von SLE-Patienten gegenüber Rheuma-Patienten dienen.

Um die erfindungsgemäßen monoklonalen Antikörper (Antihiston-Antikörper) histon-Antikörper) herzustellen, die gegen die in Körperflüssigkeiten von SLE-Patienten gerichtet sind, wurde erfindungsgemäß folgendermaßen vorgegangen (Schema I):
(1) Analyse der Histonsequenzen (mathem. Modell)
(2) Voraussage der antigenen Bereiche
(3) Synthese von Peptiden entsprechend den antigenen Bereichen. Die Peptide werden teils frei, teils gebunden am Träger (TentaGel) hergestellt.
(4a) Immunisierung von Tieren (Mäusen) mit synthetischen Peptiden entsprechend (3); die Peptide müssen trägergebunden eingesetzt werden (z.B. an TentaGele);
(4b) Immunisierung von Milzzellen in vitro mit synthetischen Peptiden entsprechend (3). Hier können freie oder trägergebundene Peptide eingesetzt werden.
(5) Isolierung der Milzzellen und Fusionierung mit Krebszellen zu Hybridomzellen; Selektion einzelner (positiver) Klone
(6) Isolierung der sezernierten Anti-Histon-Antikörper (AHA)
(7) Spezifitäts- und Aktivitätsprüfung der künstlichen AHA-s mit synthetischen Peptiden entsprechend (3) als Antigene mittels ELISA.

Um die erfindungsgemäßen antiidiotypischen Antikörper herzustellen, wurde erfindungsgemäß wie folgt vorgegangen (Schema II):
(1.1) Auswahl des Antigens:
   Das Antigen ist z.B. das gegen die Histonpeptide H1 (187 - 211) und H2B (1 - 35) gerichtete Epitop auf den Autoantikörper im Serum von SLE-Patienten oder
(1.2) das entsprechende Epitop auf den monoklonalen Antikörpern, welche gegen diese Peptide/Peptidkombination hergestellt wurden.
(2) Gewinnung des Antigens/der Antigene;
   (2.1) Die Antikörperfraktion des SLE-Serums wird nach dem üblichen Verfahren angereichert;
      (2.2.1) Aus der angereicherten Antikörperfraktion des SLE-Serums werden die Autoantikörper, welche die nach (1) definierten Epitope besitzen, durch affinitätschromatographische Verfahren selektiv isoliert. Dazu werden die unter (1) definierten Peptide mittels geeigneter Verfahren an geeignete Trägermaterialien chemisch oder adsorptiv gebunden.
         Alternativ lassen sich auch die Peptide an gegeeigneten Trägermaterialien, z.B. Tenta-Gelen synthetisieren. Man kann also die reicherte Antikörperfraktion des SLE-Serums zuerst über eine Säule mit Träger-H1 (187-211)-Konjugat schicken, waschen und die an das Konjugat gebundenen Autoantikörper mit geeigneten Methoden eluieren. Diese körperfraktion wird dann in einem zweiten Schritt über eine Säule mit einem Träger-H2B (1 - 35)-Konjugat geleitet. Die interessierenden Autoantikörper mit Doppelspezifität (oder Kreuzspezifität) werden dann festgehalten und können nach dem Waschen der Säule mit geeigneten Methoden eluiert werden. Man kann die Reihenfolge der Affinitätsschritte auch vertauschen, also zuerst Träger-H2B-(1 - 35) und dann Träger-H1C einsetzen.
      (2.2.2) Die monoklonalen Antikörper, welche das doppeltspezifische Epitop entsprechend (1.2) besitzen, sind nach Schema I (6) isoliert und gereinigt worden.
3. Immunisierungsverfahren
   (3.1) In vivo-Immunisierung
      Die unter (2) gewonnenen Autoantikörper bzw. monoklonalen Antikörper werden in der üblichen Weise zur Immunisierung geeigneter Versuchstiere eingesetzt. Sie können frei in Kombination mit geeigneten Adjuvantien eingesetzt werden oder an geeignete Träger gekoppelt werden, z.B. TentaGele.
   (3.2) In vitro-Immunisierung
      Die unter (2) gewonnenen Antikörper können auch benutzt werden, um Milzzellen geeigneter Versuchstiere in vitro nach den bekannten Verfahren zu immunisieren.
(4) Isolierung der antiidiotypischen Antikörper produzierenden Milzzellen und Fusion mit geeigneten Krebszellen zu Hybridomzellen.
(5) Selektion und Anzucht einzelner Klone.
(6) Isolierung und Reinigung der monoklonalen antiidiotypischen Antikörper.

Es ist auch denkbar, statt (3) aus dem Blut von SLE-Patienten (oder Tieren mit Autoimmunkrankheiten) B-Lymphocyten zu isolieren, mit Tumorzellen zu fusionieren und aus den erhaltenen Hybridomzellen diejenigen Klone zu isolieren, welche die unter (1) definierte Spezifität besitzen. Die Identifizierung dieser Klone geschieht mittels üblicher Tests, z.B. ELISA-Technik, unter Verwendung der erfindungsgemäßen Peptide/Peptid-Kombinationen.

Es ist klar, daß die Bestimmung der Konzentration der Autoantikörper (Anti-Histon-Antikörper)von SLE - Patienten nicht auf ELISA-Verfahren beschränkt ist.

Die AHA-Konzentration kann auch mittels Radioimmuno-Assay (RIA) durch Verwendung radioaktiv markierter N-terminaler Peptide von H2B und C-terminaler Peptide von H1 oder mittels Fluoreszenzimmuno-Assay durch Verwendung fluoreszierend markierter N-terminaler Peptide von H2B und C-terminaler Peptide von H1 bestimmt werden. Es ist dem Fachmann klar, daß die Nachweise und Konzentrationsbestimmungen für die AHA außer in Seren auch in anderen Körperflüssigkeiten und -bestandteilen durchgeführt werden können, z.B. im Urin.

Erfindungsgemäß zeigt sich, daß antigene Determinanten der Histone H1 und H2 sowohl mittels künstlich hergestellter monoklonaler Antikörper als auch mittels humaner pathogener Autoantikörper charakterisiert werden können. Um die Antigenität der sehr konservativen und schwach immunogenen Histone zu verbessern, wurden gereinigte Histonklassen bzw. ausgewählte synthetische Peptide an verschiedene Träger gekoppelt.

Aus der in vivo Immunisierung mit glutaraldehyd - vernetzten Histonkomplexen ging ein IgM-Antikörper (1/A8/B1) hervor, der gegen Konformationsantigene gerichtet ist. Die in vivo Immunisierung mit an Eupergit C gekoppelten Histon H1 ergab drei weitere monoklonale IgM-Antikörper: 1/H4/C3 (IgG₂ₐ, 1/H4/C6 (IgG₂ₐ) und 1/H4/C10 (IgG₂ₐ), alle mit Kappa-Spezifität der leichten Kette. Das Epitop der drei monoklonalen Antikörper lag im C-Terminus von H1 (187 - 211). Die Kreuzreaktion der Antikörper mit dem T-Terminus von H2B (22 - 35) ist auf Sequenz- und Ladungshomologien beider terminaler Histonbereiche zurückzuführen. Zwei N-terminale Peptide aus H2B, gekoppelt an Eupergit , wurden zur in vivo Immunisierung eingesetzt.

Als Antigene wurden freie Histone, freie Peptide und Träger gekoppelte Peptide eingesetzt. Die in vitro Immunisierung mit freiem Histon H1 ergab einen IgG₂ₐ Antikörper mit Kappa-Kette, dessen Epitop auch der C-Terminus von H1 ist.

Erfindungsgemäß konnten TentaGele als neues synthetisches Trägermaterial erfolgreich zur in vitro Immunmisierung eingesetzt werden. Die TentaGele stellen eine neue Klasse von aufgepfropften copolymeren Partikeln dar, deren Polystyrolkern von "Bürstensaumähnlichen" Polyoxyethylententakeln umgeben ist. Diese Träger können in einem "Einschrittsverfahren" nach der Peptidsynthese sofort zur in vitro Immunisierung eingesetzt werden . Die TentaGele zeichnen sich durch eine sehr gute Biokompatibilität, chemische Inertheit, verbesserte Hydrophileigenschaften und nicht zuletzt durch die optimale Exposition uniformer haptener Strukturen zum Kontakt mit immunkompetenten Zellen aus.

Die erhaltenen monoklonalen Antikörper wurden sowohl in verschiedenen immunologischen Testsystemen, wie Immundiffusion, Hämagglutination , Dot Blot und unterschiedlichen ELISA-Testsystemen verwendet, als auch nach Kopplung mit Fluoreszenzisothyocyanat (FITC) und Meerettichperoxidase (HRP) in der Durchflußcytometrie und im Western Blot eingesetzt.

## Patentansprüche

1. Monoklonale Antikörper, die die Spezifität besitzen, von den nachstehenden Aminosäuresequenzen (im Einbuchstabencode) 
(1) K P K A A  K P K A A  K P K A A  K P K K A
    A P K K K,
(2) P E P A K  S A P A P  K K G S K  K A V T K
    A Q K K D  G K K R K  R S E K E,
(3) S Y S V Y  V Y K V L  K Q V H P  D T G I S
    S K A M G  I M N S F  V N D I F  E R I A G E.
 sowohl die Sequenz (1) als auch die Sequenz (2) oder sowohl die Sequenz (1) als auch die Sequenz (3) zu erkennen.

2. Monoklonale Antikörper nach Anspruch 1 zur Herstellung von antiidotypischen Antikörpern, die in Körperflüssigkeiten von SLE-Patienten Autoantikörper erkennen.

3. Monoklonale Antikörper nach Anspruch 1 zur Herstellung von antiidiotypischen Antikörpern zur Diagnose und/oder Therapie von SLE

4. Monoklonaler Antikörper nach Anspruch 1 zur Herstellung von antiidiotypischen Antikörpern zur Elution oder zur Bestimmung der Konzentration von Autoantikörpern von SLE-Patienten.

5. Monoklonale Antikörper nach Anspruch 1,
**dadurch gekennzeichnet**, daß die Aminosäuresequenzen durch Aminosäure-Insertion und/oder Aminosäure-Deletion und/oder Aminosäure-Substitution modifiziert sind, wobei jeweils die antigenen oder immunogenen Eigenschaften im wesentlichen unverändert erhalten sind.

6. Monoklonale Antikörper nach Anspruch 5,
**dadurch gekennzeichnet,** daß die Aminosäuresequenzen mit Peptidbindungen, wie
-CON(CH₃)-, -CH₂-CH₂-, -CO-CH₂-.
modifiziert sind.

7. Monoklonaler Antikörper nach Anspruch 1,
**dadurch gekennzeichnet**, daß die Aminosäuresequenz (1) den Sequenzabschnitt 187 - 211 vom Histon H1 (C-Terminus) oder Teilen hiervon mit wenigstens einer antigenen oder immunogenen Determinante (Epitop) umfaßt.

8. Monoklonaler Antikörper nach Anspruch 1,
**dadurch gekennzeichnet**, daß die Aminosäuresequenz (2) den Sequenzabschnitt 1 - 35 von Histon H2B (N-Terminus) oder Teilen hiervon mit wenigstens einer antigenen oder immunogenen Determinante (Epitop) umfaßt.

9. Monoklonaler Antikörper nach Anspruch 1,
**dadurch gekennzeichnet,** daß die Aminosäuresequenz (3) den Sequenzabschnitt 36 - 76 von Histon H2B oder Teilen hiervon mit wenigstens einer antigenen oder immunogenen Determinate (Epitop) umfaßt.

## Claims

1. Monoclonal antibodies, which have the specificity to recognise from the following amino acid sequences (in single-letter code) : 
(1) K P K A A  K P K A A  K P K A A  K P K K A
    A P K K K,
(2) P EP A K S A P A P K K G S K  K A V T K
    A QK K D  G K K R K R S E K E,

(3) S Y S V Y  V Y K V L  K Q V H P  D T G I S
 S K A M G  I M N S F  V N D I F  E R I A G E.
 both sequence (1) and sequence (2), or both sequence (2) and sequence (3).

2. Monoclonal antibodies according to claim 1 for producing anti-idiotypical antibodies which recognise autoantibodies in body fluids of SLE patients.

3. Monoclonal antibodies according to claim 1, for producing anti-idiotypical antibodies for diagnosis and/or therapy of SLE.

4. Monoclonal antibody according to claim 1, for producing anti-idiotypical antibodies for elution or for determination of the concentration of autoantibodies of SLE patients.

5. Monoclonal antibodies according to claim 1,
characterised in that
the amino acid sequences are modified by amino acid insertion and/or amino acid deletion and/or amino acid substitution, the respective antigen or immunogenic properties being substantially retained in each case.

6. Monoclonal antibodies according to claim 5,
characterised in that
the amino acid sequences are modified with peptide bonds such as
-CON(CH₃)-, -CH₂-CH₂-, -CO-CH₂-.

7. Monoclonal antibody according to claim 1, characterised in that the amino acid sequence (1) comprises the sequence section 187 - 211 of histon H1 (C terminus) or portions thereof with at least one antigen or immunogenic determinant (Epitop).

8. Monoclonal antibody according to claim 1,
the amino acid sequence (2) comprises the sequence section 1 - 35 of histon H2B (N terminus) or portions thereof with at least one antigen or immunogenic determinant (Epitop).

9. Monoclonal antibody according to claim 1,
characterised in that
the amino acid sequence (3) comprises the sequence section 36 - 76 of histon H2B or portions thereof with at least one antigen or immunogenic determinant (Epitop).

## Revendications

1. Anticorps monoclonaux, qui présentent la spécificité leur permettant de reconnaître, parmi les séquences d'acides aminés suivantes (qui utilisent le code à une lettre) 
(1) K P K A A  K P K A A  K P K A A  K P K K A
    A P K K K,
(2) P E P A K  S A P A P  K K G S K  K A V T K
    A Q K K D  G K K R K  R S E K E,
(3) S Y S V Y  V Y K V L  K Q V H P  D T G I S
    S K A M G  I M N S F  V N D I F  E R I A G E.
 tant la séquence (1) que la séquence (2), ou encore tant la séquence (1) que la séquence (3).

2. Anticorps monoclonaux selon la revendication 1, pour préparer des anticorps anti-idiotypiques, qui, dans les fluides corporels de patients présentant un lupus érythémateux disséminé (LED), reconnaissant les auto-anticorps.

3. Anticorps monoclonaux selon la revendication 1, pour préparer des anticorps anti-idiotypiques pour le diagnostic et/ou le traitement du LED.

4. Anticorps monoclonaux selon la revendication 1, pour préparer des anticorps anti-idiotypiques destinés à l'élution ou à la détermination de la concentration d'auto-anticorps de patients présentant un LED.

5. Anticorps monoclonaux selon la revendication 1, caractérisés en ce que les séquences d'acides aminés sont modifiées par insertion d'acides aminés et/ou par délétion d'acides aminés et/ou par substitution d'acides aminés, les propriétés antigéniques ou immunogéniques étant dans chaque cas maintenues essentiellement inchangées.

6. Anticorps monoclonaux selon la revendication 5, caractérisés en ce que les séquences d'acides aminés sont modifiées par des liaisons peptidiques telles que
-CON(CH₃)-, -CH₂-CH₂-, -CO-CH₂-

7. Anticorps monoclonaux selon la revendication 1, caractérisés en ce que la séquence d'acides aminés (1) comporte le fragment de séquence 187-211 de la histone H1 (C-terminale) ou de parties de cette dernière, avec au moins un déterminant antigénique ou immunogénique (épitope).

8. Anticorps monoclonal selon la revendication 1, caractérisé en ce que la séquence d'acides aminés (2) comporte le fragment de séquence 1-35 de la histone H2B (N-terminale) ou de parties de cette dernière, avec au moins un déterminant antigénique ou immunogénique (épitope).

9. Anticorps monoclonal selon la revendication 1, caractérisé en ce que la séquence d'acides aminés (3) comporte le fragment de séquence 36-76 de la histone H2B ou de parties de cette dernière, avec au moins un déterminant antigénique ou immunogénique (épitope).
